# EUROPÄISCHE PATENTSCHRIFT

(11) **EP 1 989 117 B1**
(45) Veröffentlichungstag und Bekanntmachung des Hinweises auf die Patenterteilung: **13.06.2012**
(21) Anmeldenummer: 06840986.1
(22) Anmeldetag: 15.12.2006
(51) Int. Cl.: B65D 1/09, A61J 1/06

(54) **BEHÄLTER SOWIE VORRICHTUNG ZUM HERSTELLEN DESSELBEN**
CONTAINER AND APPARATUS FOR PRODUCING THE SAME
RECIPIENT ET DISPOSITIF POUR LE FABRIQUER

(30) Priorität: 01.03.2006 DE 102006009766; 02.03.2006 US 366022
(43) Veröffentlichungstag der Anmeldung: 12.11.2008
(73) Patentinhaber: Hansen, Bernd, 74429 Sulzbach-Laufen (DE)
(72) Erfinder: Hansen, Bernd, 74429 Sulzbach-Laufen (DE)
(74) Vertreter: Bartels, Martin Erich Arthur
(86) Internationale Anmeldenummer: PCT/EP2006/012105
(87) Internationale Veröffentlichungsnummer: WO 2007/101466

(56) Entgegenhaltungen:
- WO-A-01/89940
- DE-C- 259 204
- FR-A- 1 283 152
- US-A- 4 948 356
- US-A1- 3 993 223
- US-A1- 2004 182 883

## Beschreibung

Die Erfindung betrifft einen Behälter, insbesondere eine hermetisch verschlossene Ampulle, umfassend einen hohlen Aufnahmekörper zur Aufnahme eines Abgabemediums, eine am Aufnahmekörper ausgebildete Abgabeöffnung zur Abgabe des Abgabemediums aus dem Aufnahmekörper und ein Verschlussteil, das außerhalb des Aufnahmekörpers eine die Abgabeöffnung freihaltende Hohlkammer aufweist und ein Kopfteil des Behälters bildet, wobei das Verschlussteil mit dem Aufnahmekörper über eine Trennstelle lösbar verbunden ist und im Bereich der Hohlkammer einander gegenüberliegende Wandteile aufweist.

Dahingehende Behälter der genannten Art dienen insbesondere dazu, in dem hohlen Aufnahmekörper Abgabemedien der verschiedensten Art zu bevorraten, beispielsweise in Form medizinischer Lösungen, Suspensionen oder halbfesten Zubereitungen, wie Gelen. Um eine Abgabeöffnung des Aufnahmekörpers rasch freigeben zu können, ist ein das Kopfteil des Behälters bildendes Verschlußteil vorgesehen, das in einer sog. Twist-Off-Bewegung (Abdrehbewegung) als Knebelverschlußteil über eine vorgegebene Trennstelle vom sonstigen Aufnahmekörper abtrennbar ist.

Damit die im Aufnahmekörper vorgesehene Abgabeöffnung während des Formvorganges für den Behälter für die Trennstelle nicht ungewollt verschlossen wird, weist das Verschlußteil eine Hohlkammer auf, die fluid- oder medienführend in den Aufnahmekörper über die Abgabeöffnung übergeht und deren Seitenrand umgrenzt, der im übrigen nach außen hin von den Wandteilen des Behälters begrenzt ist. Wird beim Öffnen des Knebelverschlusses mittels der Twist-Off- oder Abdrehbewegung das Verschlußteil über die Trennstelle vom sonstigen Aufnahmekörper abgetrennt, sorgt auch insoweit die Hohlkammer dafür, dass die Abgabeöffnung mit ihrer ursprünglichen Ausgangsgestalt frei bleibt und nicht etwa begrenzende Wandteile des Behälters derart gegeneinander verschoben werden, dass ungewollt es zu einem Verschluß der Abgabeöffnung käme.

So betreffen die US 3 993 223 A1, DE 259 204 C und FR 1 283 152 A Behälterlösungen mit einer pipettenartig sich verjüngenden Abgabeöffnung, um dergestalt ein ungewolltes Austreten des Abgabemediums aus dem Abgabebehälter vermeiden zu helfen. Allein bei dem dahingehenden Abtrennen oder Abscheren des jeweiligen Kopf Verschlussteiles zwecks Freigabe der Abgabeöffnung, kann es aufgrund der an die Abgabeöffnung angrenzenden Wandteile zu deren Verschiebung kommen, was ungewollt zu dem aufgezeigten Verschluss der Abgabeöffnung führen kann.

Um eine ungehinderte Abgabe des im Aufnahmekörpers aufgenommenen Abgabemediums sicherstellen zu können, ist der freie Querschnitt der Abgabeöffnung entsprechend groß zu dimensionieren und ebenso der freie Eintrittsquerschnitt der genannten Hohlkammer. Letzteres führt dann aber zu dem Nachteil, dass im Aufnahmekörper bevorratetes Medium in die Hohlkammer übertritt und dann nicht mehr zurückfließen kann, insbesondere wenn das in der Hohlkammer bevorratete Fluidmedium aufgrund seiner Oberflächenspannung in der Hohlkammer festgehalten ist. Verbleibt aber ein Teil des Abgabemediums in der Hohlkammer, geht dieses für einen Entnahmevorgang aus dem Behälter verloren, was insbesondere dann von Nachteil ist, wenn es bei der Abgabe auf eine genaue Dosierung ankommt oder das Abgabemedium an sich sehr teuer ist oder wenn es bei der Abgabe von Suspensionen auf genaue Mischungsverhältnisse und Konzentrationen für die spätere Anwendung ankommt.

Zwar schlägt die DE 102 02 907 A1, um einem dahingehenden Wirkstoffverlust vorzubeugen, vor, einen hermetisch verschlossenen Behälter mit verbessertem Verschluß zu versehen; allein die dahingehend vorgeschlagenen Maßnahmen können immer noch zu einem Verlust an Wirkstoff in der Hohlkammer des als Kopfteil ausgebildeten Verschlußteils führen. Der bekannte Behälter weist als Teil einer Abgabeöffnung ein Ausgabemundstück auf sowie einen hohlen Verschlußabschnitt mit einem erhöhten Bereich. Der Verschlußabschnitt ist über ein zerreißbares Band als Trennstelle mit dem Ausgabemundstück verbunden.

Der erhöhte Bereich im hohlen Verschlußabschnitt vermindert teilweise das Zurückbleiben von Flüssigkeitstropfen auf der Innenseite des Verschlußabschnittes, wobei das Ausgabemundstück eine geradlinig nach innen und oben zusammenlaufende Wand aufweist, die bewirken soll, dass Flüssigkeitstropfen oder Flüssigkeitsteile im Verschlußabschnitt und im Ausgabemundstück nach unten in den Körperabschnitt des Behälters mit dem hohlen Aufnahmekörper zurückströmen. Folglich wird dann auch keine Flüssigkeit mehr verspritzt, wenn der Verschlußabschnitt entlang des zerreißbaren Bandes als Trennstelle vom Ausgabemundstück als Teil der Abgabeöffnung abgetrennt wird.

Trotz dieser Maßnahmen ist es insbesondere bei Fluidmedien mit hoher Oberflächenspannung nicht ausgeschlossen, dass diese im verbleibenden Hohlkammervolumen sich absetzen und dann für die Entnahme nicht zur Verfügung stehen und durch die zwingend vorgesehenen geradlinig verlaufenden Behälterwände im Bereich des Ausgabemundstücks ist die freie Gestaltung von Behältergeometrien eingeschränkt. Der genannte bekannte Behälter läßt sich im Rahmen eines üblichen Blasform-, Füll- und Siegelver fahrens erhalten, ebenso wie der nachstehend beschriebene erfindungsgemäße Behälter.

Des weiteren ist durch die US 2004/182883 A1 ein gattungsgemäßer hermetisch verschlossener Behälter bekannt, insbesondere eine hermetisch verschlossene Ampulle, mit einem hohlen Aufnahmekörper zur Aufnahme eines Abgabemediums, der eine Abgabeöffnung aufweist, die außerhalb des Aufnahmekörpers, in eine die Abgabeöffnung freihaltende Hohlkammer eines, ein Kopfteil des Behälters bildenden Verschlussteils mündet, das über eine Trennstelle mit dem Aufnahmekörper im Bereich der Abgabeöffnung lösbar verschlossen ist. Ein weiterer gattungsgemäßer Behälter ist in WO 01/89940 A1 offenbart.

Unterhalb der Trennstelle und mithin außerhalb der Hohlkammer als Verschlussteil sind bei dem aus US 2004/182883 A1 bekannten Behälter unter Bildung einer Drosselstelle gegenüberliegende Wandteile des Aufnahmekörpers im Sinne einer vergleichmäßigten Verjüngung aufeinander zuverdrängt, wobei die Drosselstelle mindestens 0,1 Inch von der freigebbaren Behälteröffnung entfernt ist, um dergestalt die mögliche Menge an im Behälter bevorrateten Abgabemedium innerhalb der Hohlkammer zu reduzieren. Die bekannte Lösung baut in axialer Richtung gesehen relativ groß auf und benötigt für die Bildung der Drosselstelle eine zwischen der Hohlkammer oberhalb der Trennstelle und der Drosselstelle unterhalb der Trennstelle liegende zusätzliche Medienkammer, was insoweit zu einem zusätzlichen Herstellaufwand führt. Bei der bekannten Lösung verbleibt also zumindest teilweise das Abgabemedium innerhalb der genannten Hohlkammer oberhalb der Trennstelle mit der Behälteröffnung, so dass es insoweit auch zu Verlusten für das Wirk- oder Abgabemedium kommt, sofern eine Behälterentnahme durchgeführt wird.

Ausgehend von diesem Stand der Technik liegt der Erfindung die Aufgabe zugrunde, die bekannte Lösung dahingehend weiter zu verbessern, dass in der genannten Hohlkammer des Kopf- oder Verschlußteils kein Abgabemedium mehr verbleibt, das insoweit dann nicht über zwingend vorgegebene Trichtergeometrien der Abgabeöffnung in den Aufnahmekörper als eigentliches Behälterinnere zurückzuführen ist, so dass insoweit im größeren Rahmen eine freie, an Kundenvorgaben und Praktikabilitätsgesichtspunkten orientierte Behälterformgebung möglich ist. Eine dahingehende Aufgabe löst ein Behälter mit den Merkmalen des Patentanspruches 1 in seiner Gesamtheit und ein dahingehender Behälter läßt sich mit einer Vorrichtung mit der Gesamtheit der Merkmalsausgestaltung des Anspruches 8 sinnfällig herstellen.

Der erfindungsgemäße Behälter ist dadurch charakterisiert, dass einander gegenüberliegende Wandteile des Verschlussteils von der Trennstelle beabstandet in die Hohlkammer derart hinein verdrängt sind, dass der verbleibende freie Kammerquerschnitt der Hohlkammer mit seinem Aufnahmevolumen derart reduziert ist, dass das im Aufnahmekörper bevorratete Abgabemedium nicht in die Hohlkammer eindringen kann. Im Gegensatz zu der bekannten Lösung wird also kein erhöhter keil- oder kalottenartiger Bereich am freien oberen Ende des hohlen Verschlußabschnittes des Kopfteils als Hohlkammer vorgesehen, sondern vielmehr werden an den einander gegenüberliegenden Wandabschnitten, die die Hohlkammer begrenzen, diese aufeinander zu bewegt und dergestalt zur Reduzierung des freien Kammervolumens in die Hohlkammer hinein verdrängt, was insoweit zu deutlich verbesserten Volumenreduzierungen führt.

Die zur Abgabeöffnung in die Hohlkammer quer oder schräg dazu hinein verdrängten, gegenüberliegenden benachbarten Wandteile reduzieren das Hohlkammervolumen derart, dass das Abgabemedium, sei es auch in Form eines sehr dünnflüssigen Mediums, erst gar nicht in die Hohlkammer gelangen kann, sondern im hohlen Aufnahmekörper verbleibt. Insoweit stellt sich also bei der erfindungsgemäßen Lösung erst gar nicht das Problem, in die Hohlkammer eindringendes Abgabemedium über geradlinig verlaufende Schrägflächen des Ausgabemundstückes in das Behälterinnere zurückführen zu müssen, sondern vielmehr kann insoweit die Gestaltung der Abgabeöffnung sowie eines dahingehend ausgebildeten Ausgabemundstückes im weit skizzierten Rahmen frei gewählt werden, was insbesondere in axialer Richtung des Behälters kurze Längsabmessungen in der Realisierung erlaubt.

Die genannte Hohlkammer insbesondere in Kugelform dient auch dazu, dass Kunststoffmaterial bei der Formgebung über eine "Kante" ziehen zu können, um dergestalt die Wandstärke des Kunststoffs derart zu reduzieren, dass die genannte Sollbruchstelle entsteht.

Die erfindungsgemäße Vorrichtung, die dem Herstellen des genannten Behälters dient, weist mindestens zwei Formbacken zum Herstellen des Kopfteils des Behälters auf, die wiederum Formausnehmungen aufweisen für die Hohlkammer und für die die Hohlkammer begrenzenden Wandteile des Verschlußteils. Dahingehende Vorrichtungen sind in einer Vielzahl von Ausführungsformen auf dem Markt frei erhältlich und erlauben die Ausführung eines sog. Blasform-, Füll- und Siegelverfahrens, wie es in der Fachwelt beispielsweise unter der Markenbezeichnung "bottelpack^{®}" bekannt geworden ist.

Ausgehend von diesen bekannten Vorrichtungen zeichnet sich die erfindungsgemäße Vorrichtung dadurch aus, dass innerhalb der die Hohlkammer betreffenden Formausnehmungen ansteuerbare Formteile vorhanden sind, die einander zugewandt in einer eingefahrenen Stellung die vollständige Hohlkammerbildung ermöglichen und in einer ausgefahrenen Stillung die einander benachbarten Wandteile der Hohlkammer formend unter Verringerung des freien Kammervolumens aufeinander zu bewegen. Die dahingehenden, vorzugsweise als pneumatisch antreibbare Arbeitsstempel ausgebildeten Formteile lassen sich insoweit platzsparend innerhalb der jeweiligen Formbacken mit ihren Formausnehmungen unterbringen und zeit- sowie prozeßsicher ansteuern. Durch die Integration der zusätzlichen Formteile innerhalb der Formbacken ist darüber hinaus eine platzsparende Unterbringung gewährleistet sowie eine funktionssichere Ausgestaltung, die sich im übrigen insoweit kostengünstig realisieren läßt.

Weitere vorteilhafte Ausführungsformen sind Gegenstand der weiteren Unteransprüche.

Im folgenden werden der erfindungsgemäße Behälter sowie die Vorrichtung anhand je eines Ausführungsbeispiels nach der Zeichnung näher erläutert. Dabei zeigen in prinzipieller und nicht maßstäblicher Darstellung die
- Fig.1: in Vorderansicht einen Behälterverbund aus fünf Einzelbehältern, wobei in Blickrichtung auf die Fig.1 gesehen der äußerst rechte Behälter eine Ausführung nach dem Stand der Technik betrifft;
- Fig.2: eine Seitendarstellung auf den Verbund nach der Fig.1;
- Fig.3: in vergrößerter Darstellung das in Blickrichtung auf die Fig.1 äußerst links dargestellte Verschlußteil innerhalb des dort mit "X" bezeichneten Kreisausschnittes;
- Fig.4: die stirnseitige Ansicht von Elementen einer Formbakke, wobei die Formbackenteile dargestellt sind zur Herstellung der Behältergeometrie nach der Fig.3 und der eigentliche Behälter ist vom Formwerkzeug her der einfacheren Darstellung wegen in Fig.4 nicht gezeigt;
- Fig.5: in perspektivischer Darstellung einen um 180° gedrehten Teilausschnitt betreffend das Formwerkzeug nach der Fig.4,einmal mit ausgefahrenem und einmal mit eingefahrenem Arbeitsstempel;
- Fig.6: einen Längsschnitt durch das Formwerkzeug nach der Fig.4 für eine Herstellform des Behälters.

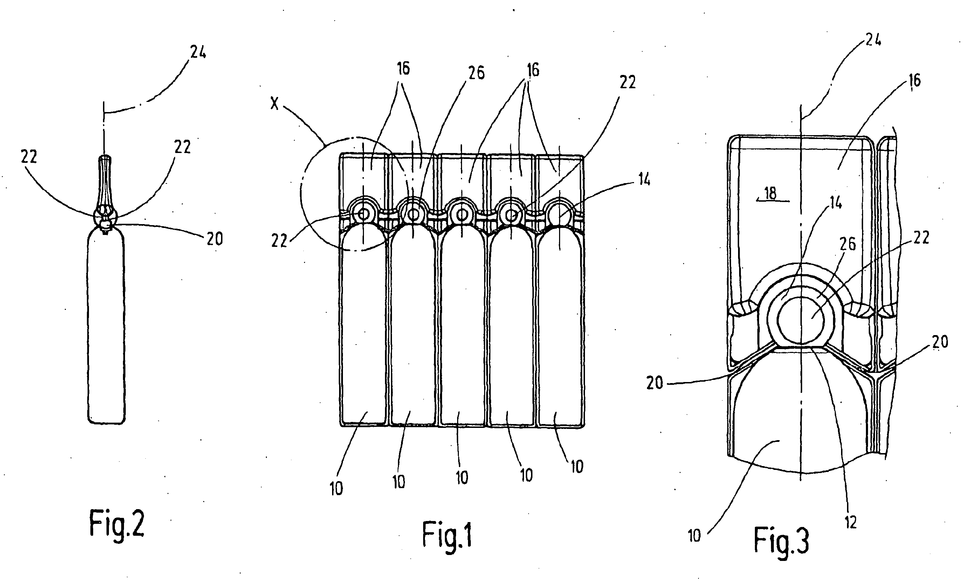

Der erfindungsgemäße Behälter ist insbesondere in Form einer hermetisch verschlossenen Ampulle ausgebildet. Ein Verbund von fünf dahingehenden Behältern ist in der Fig.1 dargestellt, wobei die einzelnen Behälter längs ihres jeweils aneinanderstoßenden Längsrandes voneinander unter Bildung eines Einzelbehälters separierbar sind. Es lassen sich nahezu beliebige Reihen an herzustellenden Behältern erzeugen sowie auch einzelne Behälter.

Die genannten Behälter sind aus einem formbaren Kunststoffmaterial hergestellt, beispielsweise bestehen diese aus Polyamid oder einem Polyethylen-Werkstoff. Des weiteren besteht die Möglichkeit, dahingehende Behältererzeugnisse im Co-Extrusionsverfahren mit einem mehrschichtigen Wandaufbau zu erzeugen oder den Kunststoff, um dessen Impermeabilität zu erhöhen, mit einer Beschichtung zu versehen, beispielsweise den Behälter außenseitig zu bedampfen. Vorzugsweise werden jedenfalls Materialien eingesetzt, die für ein Blasform-, Füll- und Siegelverfahren, wie es beispielhaft unter der Markenbezeichnung "bottelpack^{®}" der Fachwelt bekannt geworden ist, gut verwendbar sind.

Jeder Behälter weist einen hohlen, blasgeformten Aufnahmekörper 10 auf, der zur Aufnahme eines nicht näher dargestellten Abgabemediums dient, unter anderem in Form steril abgefüllter, medizinischer Flüssigkeiten, Suspensionen, Gelen und dergleichen mehr. Wie insbesondere die Fig.3 zeigt, ist der Aufnahmekörper 10 zur Abgabe des bevorrateten Abgabemediums mit einer Abgabeöffnung 12 versehen, die außerhalb des Aufnahmekörpers 10 in eine, die Abgabeöffnung 12 freihaltende Hohlkammer 14 eines ein Kopfteil 16 des Behälters bildenden Verschlußteils 18 mündet, das über eine Trennstelle 20 mit dem Aufnahmekörper 10 im Bereich der Abgabeöffnung 12 lösbar verbunden ist. Die dahingehende Trennstelle 20 kann durch ein in der Wandstärke reduziertes Kunststoff-Wandteil gebildet sein, das sich zwischen Kopfteil 16 und Aufnahmekörper 10 erstreckt.

Die dahingehende Trennstelle 20 ist jedenfalls derart ausgebildet, dass das Kopf- oder Verschlußteil 16,18 in der Art eines Knebelverschlusses von Hand ohne weiteres von dem Aufnahmekörper 10 abdrehbar ist (Twist-Off-Verschluß), um dergestalt die Freigabe der Abgabeöffnung 12 für einen Entnahmevorgang des Abgabemediums freizulegen. Der dahingehende Separierungsvorgang ist im Stand der Technik bei einer Vielzahl von Ausführungsformen, wie sie auf dem Markt frei erhältlich sind, bekannt, so dass an dieser Stelle hierauf nicht mehr näher eingegangen wird.

In Blickrichtung auf die Fig.1 gesehen ist äußerst rechts ein Behälter 10 dargestellt, der noch mit einer konventionellen Hohlkammer-Lösung ausgestattet ist. Die dahingehende Hohlkammer 14 ist in der Art einer Vollkugel ausgebildet und stellt mit ihrem unteren Umfassungsrand sicher, dass sowohl während des Formvorganges für den Behälter als auch später beim Abtrennen des Kopfteils 16 die Abgabeöffnung 12 sich nicht ungewollt verschließt, sondern für den gewünschten Entnahmevorgang offen bleibt.

Dank der Kugelform für die Hohlkammer 14 ist sichergestellt, dass das eingesetzte Kunststoffmaterial im Formwerkzeug über eine Art Kante gezogen werden kann, um dergestalt das Kunststoffmaterial unter Bildung der Sollbruchstelle 20 zu reduzieren.

Nun kann aber nicht ausgeschlossen werden, dass das im Aufnahmekörper 10 bevorratete Abgabemedium in die Hohlkammer 14 gelangt und dort begünstigt durch die jeweilige Oberflächenspannung des Abgabemediums verbleibt mit der Folge, dass das in der Hohlkammer 14 aufgenommene Abgabemedi um für den späteren Abgabevorgang über den eigentlichen hohlen Aufnahmekörper 10 nicht mehr zur Verfügung steht. Die dahingehende Fehlmenge ist insbesondere dann nicht tolerierbar, wenn es auf eine hochgenaue Dosierung ankommt, das Abgabemedium an sich sehr teuer ist und/oder bei im Aufnahmekörper aufgenommenen Mischungen und Suspensionen die vorgeschriebene oder gewünschte Konzentration eine wichtige Rolle spielt.

Demgegenüber zeigen in Blickrichtung auf die Fig.1 gesehen die sich an die konventionelle Lösung anschließenden, links angeordneten vier Behälter die erfindungsgemäße Behälterlösung, bei der zur Reduzierung des freien Volumens der Hohlkammer 14 die einander gegenüberliegenden Wandteile 22 des Verschlußteils 18 in die Hohlkammer 14 hinein verdrängt sind, um dergestalt zu vermeiden, dass das im Aufnahmekörper 10 bevorratete Abgabemedium in die Hohlkammer 14 überhaupt noch eindringen kann.

Vorzugsweise ist dabei vorgesehen, dass gemäß der Darstellung nach den Figuren die verdrängten Wandteile 22 des Verschlußteils 18 sich quer zu der Abgabeöffnung 12 erstrecken, so dass dergestalt in einem weit gezogenen Rahmen sich eine relativ große Anlagefläche für die gegenüberliegenden Wandteile 22 ergibt, die insoweit auch quer zur Längsachse 24 des Behälters (vergleiche Fig.2 und 3) gegeneinander verdrängt in Anlage miteinander sind. In Abhängigkeit der Ausführungsform wäre es aber auch möglich, die zu verdrängenden Wandteile 22 in einem schrägen Winkel (nicht dargestellt) gegensinnig aufeinandertreffen zu lassen.

Dadurch, dass beide gegenüberliegenden Wandteile 22 gleichmäßig aufeinander zu bewegt werden, ist ein günstiges Nachfließverhalten für das sonstige Kunststoffmaterial geschaffen, so dass Spannungsspitzen beim Formgebungsprozeß vermieden sind und somit etwaige Versagensstellen. Auch wurde durch die gleichförmige Verdrängungsbewegung beider Wandteile 22 für eine Hohlkammer 14 sichergestellt, dass der sensible Bereich der Abgabeöffnung nicht tangiert wird, um insbesondere sicherzustellen, dass der freie Umfassungsrand der Abgabeöffnung vom Querschnitt her erhalten bleibt, um dergestalt später einen reibungslosen Entnahmevorgang sicherstellen zu können. Für eine sinnfällige Prozeßsteuerung ist es von Vorteil, dass die einander benachbart gegenüberliegenden verdrängten Wandteile 22 sich innerhalb der Hohlkammer 14 des Verschlußteils 18 berühren; allein für die Funktion der Erfindung würde es aber auch genügen, die Wandteile 22 jedenfalls insoweit aufeinander zu zu bewegen, dass der verbleibende freie Kammerquerschnitt der Hohlkammer 14 mit seinem Aufnahmevolumen derart reduziert ist, dass das Abgabemedium nicht in die Hohlkammer 14 aufgrund seiner Oberflächenspannung gelangen kann.

Wie die Fig.1 und 3 des weiteren zeigen, lassen die verdrängten, einander berührenden Wandteile 22 innerhalb der Hohlkammer 14 ein ringförmiges Kammersegment 26 frei und die dahingehende Ausgestaltung stellt bezüglich des Nachfließverhaltens des Kunststoffmaterials ebenso ein Optimum dar wie für die eigentliche Formgebung und das Abhaltevermögen für das Abgabemedium. Unabhängig hiervon ist es aber möglich, bei besonderen Behältergeometrien oder zur Realisierung spezieller Luer-Lock-Formen die aufeinander zu verdrängten, gegenüberliegenden Wandteile anders zu definieren (nicht näher dargestellt). Der genannte Ringraum in Form des ringförmigen Kammersegmentes 26 mündet in Blickrichtung auf die Figuren gesehen von oben her in die Abgabeöffnung 12 ein und erlaubt insoweit einen Druckausgleich bezogen auf die Situation des Aufnahmekörpers 10, so dass insoweit auch nicht ungewollt, beispielsweise über Vakuum-Effekte, das Abgabemedium aus dem Aufnahmekörper 10 in Richtung der Hohlkammer 14 ansaugbar wäre, was auch für den Fall gilt, dass die Abtrennung über die Trennstelle 20 vollzogen wird.

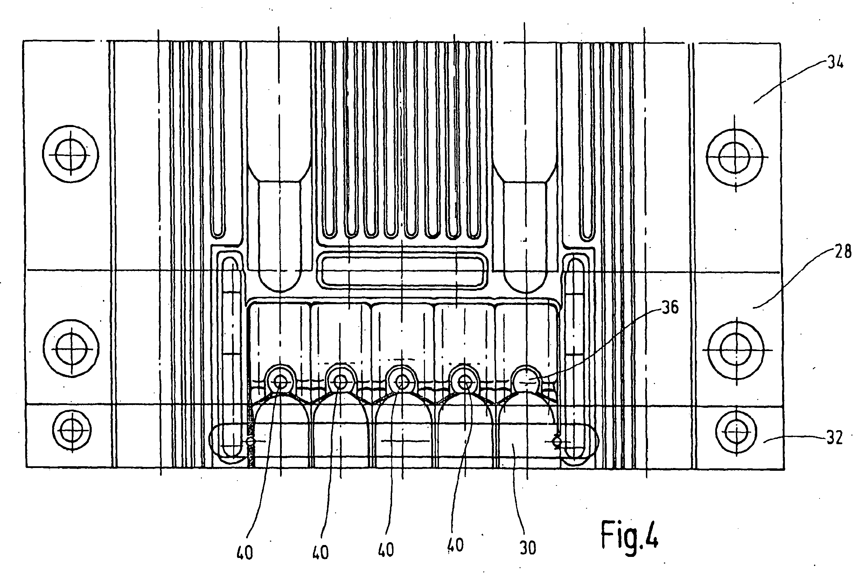

Die erfindungsgemäße Vorrichtung zum Herstellen des Behälters nach einem der Fig.1 bis 3 ist gemäß den Darstellungen nach den Fig.4ff näher erläutert. Die erfindungsgemäße Vorrichtung weist mindestens zwei Formbacken 28 auf zum Herstellen des Kopfteils 16 des Behälters. Gemäß der Darstellung nach der Fig.4 ist dort nur hälftig eine der beiden Formbacken 28 dargestellt und zum Öffnen und Schließen der Form bewegt sich eine zweite, nicht näher dargestellte Formbacke mit korrespondierenden Formausnehmungen 30 von der gezeigten Formbacke 28 nach der Fig.4 weg bzw. auf diese zu. Regelmäßig werden dabei beide Formbacken 28 gleichförmig aufeinander zu oder voneinander weg bewegt. Dahingehende maschinelle Formvorrichtungen sind üblich und auf dem Gebiet der Blasform-, Füll- und Siegelverfahren, wie sie beispielsweise unter der Markenbezeichnung "bottelpack^{®}" bekannt geworden sind, einschlägig in Anwendung.

Bei der in Fig.4 gezeigten Formbacke 28 ist in Blickrichtung auf die Fig.4 gesehen unterhalb noch eine weitere Formbacke 32 vorhanden, die nur teilweise dargestellt der Formgebung für den jeweiligen Aufnahmekörper 10 dient und darüberliegend ist eine Abschlußformbacke 34 angeordnet. In Abhängigkeit des gewählten Herstellverfahrens lassen sich die genannten Formbacken gemeinsam ansteuern; es besteht aber auch die Möglichkeit, einzelne Formbacken gegeneinander zu bewegen, beispielsweise zunächst ein Paar an Formbacken 28 zum Herstellen des Kopf- oder Verschlußteils 16,18 und dann erst die weiteren Paare an Formbacken 32 anzusteuern, um die gewünschten hohlen Aufnahmekörper 10 für einen Behälter zu erhalten.

Wie die Fig.4 des weiteren zeigt, sind Formausnehmungen 36 in der jeweiligen Formbacke 28 vorhanden, um die Hohlkammer 14 zu schaffen bzw. die die Hohlkammer 14 begrenzenden Wandteile des Verschlußteils 18. In Blickrichtung auf die Fig.4 gesehen ist wiederum das ganz rechts angeordnete Werkzeug für eine Behälterherstellung ausgelegt, bei der die Hohlkammer 14 als Vollhohlkugel ausgebildet wird gemäß der Behälterkonfiguration nach der ganz rechten Darstellung in Fig.1. Es besteht durchaus die Möglichkeit, innerhalb eines Formwerkzeuges freie Hohlkammerquerschnitte und solche mit reduzierten Hohlkammerquerschnitten für verschiedene Behälter miteinander zu kombinieren, sofern dies aus Praktikabilitätsgesichtspunkten heraus notwendig erscheint.

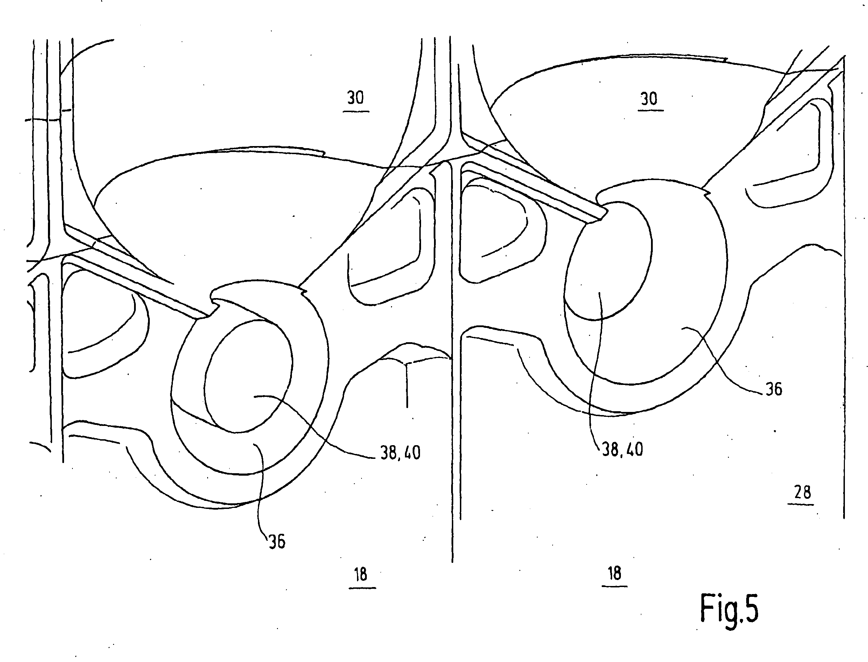

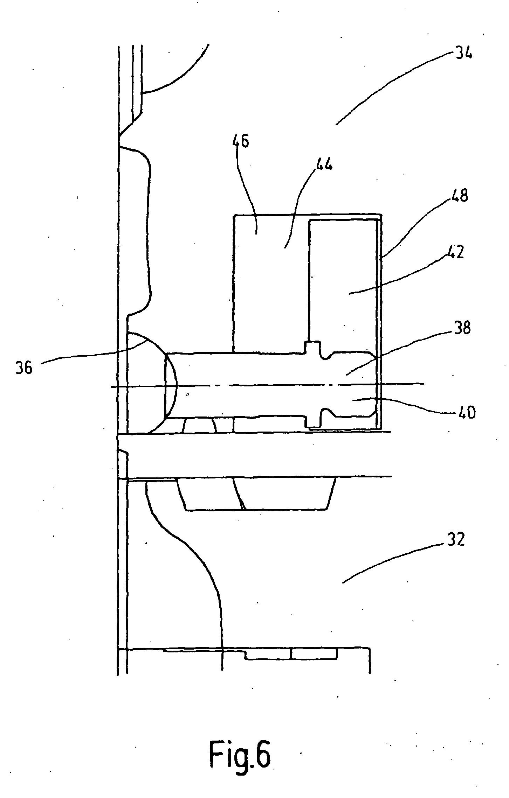

Wie des weiteren die Fig.5 und die Fig.6 zeigen, sind innerhalb der die jeweilige Hohlkammer 14 betreffenden Formausnehmungen 36 ansteuerbare Formteile 38 vorhanden, bevorzugt ausgebildet in der Art von einzelnen Arbeitsstempeln 40. Das dahingehende Formteil 38 durchgreift die kalottenartige oder halbkugelförmige Formausnehmung 36 innerhalb der jeweiligen Formbacke 28. Die dahingehenden Verhältnisse sind besonders deutlich in der Fig.5 wiedergegeben, die jedoch gegenüber der Darstellung nach der Fig.4 um 180° gedreht dargestellt ist, d.h. die Formausnehmungen 30 für die Aufnahmekörper 10 sind in Blickrichtung auf die Fig.5 gesehen oben dargestellt und die Verschlußteile 18 demgemäß gegenüberliegend nach unten gerichtet.

Zum Herstellen einer kugelförmigen Hohlkammer 14 bleibt in Blickrichtung auf die Fig.5 gesehen der rechte Arbeitsstempel 40 zurück und zum Verdrängen der Wandteile 22 ist der linke Arbeitsstempel 40 zur Bildung des erfindungsgemäßen Hohlkammervolumens nach vorne ausgefahren und ragt insoweit in die halbkugelförmige Ausnehmung 36 weit hinein und jedenfalls vorzugsweise insoweit, dass der dann dahingehend verdrängte Wandabschnitt als Wandteil 22 in Anlage kommt mit dem verdrängten Wandteil 22 des in Längsrichtung in einer Achse gegenüberliegenden, nicht näher dargestellten Arbeitsstempels 40 eines weiteren korrespondierenden Formbackenwerkzeuges.

Sofern man einen ungestörten Hohlkammerquerschnitt erreichen möchte, besteht natürlich auch die Möglichkeit, anstelle eines zurückgefahrenen zylindrischen Arbeitsstempels 40 gemäß der Darstellung nach der Fig.5 diesen völlig entfallen zu lassen und die Form in konventioneller Weise wie üblich dann dahingehend auszubilden. Hat die Hohlkammer 14 eine andere geometrische Struktur anstelle der gezeigten Kugelform, ist dann insoweit gegebenenfalls die Anpassung des freien Arbeitsquerschnitts des jeweils eingesetzten Arbeitsstempels 40 notwendig. Sofern die in Fig.4 gezeigte Formbacke 28 eine getrennt ansteuerbare Teilform betrifft, ist insoweit in Ausgestaltung eines modularen Konzeptes der Austausch der Formbacke 28 gegen eine andere ohne weiteres möglich, um dergestalt mit nur einer Formvorrichtung oder Formmaschine verschiedene Behältergeometrien - auch von der Kopf-Verschlußseite her - erzeugen zu können.

Die Darstellung nach der Fig.6 betrifft wiederum einen Längsschnitt durch das zugeordnete Formwerkzeug zu der Formbackenkonstruktion nach der Fig.4. Gemäß der Darstellung nach der Fig.6 ist dort der Arbeitsstempel 40 in einer Führungsplatte 42 aufgenommen, die innerhalb einer Steuerkammer 44 innerhalb derselben zwei Teilräume 46,48 voneinander abtrennt. Sofern die dahingehenden Teilräume 46,48 an eine Energiezufuhr angeschlossen sind, beispielsweise in Form einer Pneumatikzufuhr, läßt sich der Arbeitsstempel 40 aus seiner in der Fig.6 gezeigten inaktiven Stellung in Blickrichtung auf die Fig.6 gesehen nach links vorschieben, beispielsweise um eine aktive Position gemäß der Darstellung nach der Fig.5 einzunehmen, die den erfolgten Abschluß der Wandteilverdrängung wiedergibt.

Durch Druckbeaufschlagung in der Kammer 46 und Drucklosschalten der Teilkammer 48 läßt sich wiederum der Arbeitsstempel 40 in seine inaktive Stellung zurückverfahren, bei der die freie Stirnseite des Arbeitsstempels 40 gerade noch den Kalottenboden der Kalottenformausnehmung 36 mit abdeckt. Über nicht näher dargestellte Bewegungssensoren läßt sich die dahingehende Funktionsstellung des Arbeitsstempels 40 überwachen und an eine nicht näher beschriebene dargestellte Zentralsteuerung für eine Albeitsmaschine weiterleiten. Anstelle der Führungsplatte 42 wäre es bei einer nicht näher dargestellten Ausführungsform der erfindungsgemäßen Vorrichtungslösung auch möglich, unmittelbar den Arbeitsstempel 40 mittels eines Arbeits- oder Energiemediums anzusteuern. Auch wäre der Einsatz von Elektromagneten möglich, wie sie beispielhaft in der Magnet-Ventiltechnik seit Jahren eingesetzt werden, um eine Stempelbewegung auslösen zu können. Auch besteht die Möglichkeit, zumindest einen Teil der Arbeitsstempel 40 anstelle der Queranordnung in Schräganordnung (nicht dargestellt) verfahren zu lassen, sofern die benötigte Behältergeometrie dies verlangt.

Neben der genannten pneumatischen oder elektromagnetischen Bewegung des jeweiligen Arbeitsstempels 40 besteht auch die Möglichkeit eines rein hydraulischen oder elektrischen Antriebes. In Abhängigkeit der gewünschten Hohlkammergeometrie kann der jeweils eingesetzte Arbeitsstempel 40 zumindest an seiner freien Stirnseite auch eine ovale, sternförmige oder sonstige Formquerschnittsfläche aufweisen. Wie bereits dargelegt, kann der verfahrbare Kolben 40 auch in einer Schräganordnung verlaufen, so dass der Hohlraum 26 oberhalb der Sollbruchstelle 20 verschlossen ist. Ferner könnte in einer Fertigungsreihe für verschiedene zusammenhängende Behälter in Abhängigkeit der Geometrie des jeweils eingesetzten Arbeitsstempels unterschiedliche Hohlkammerformen mit den zugeführten Wandteilen hergestellt werden.

## Patentansprüche

1. Behälter, umfassend:
- einen hohlen Aufnahmekörper (10) zur Aufnahme eines Abgabemediums,
- eine am Aufnahmekörper (10) ausgebildete Abgabeöffnung (12) zur Abgabe des Abgabemediums aus dem Aufnahmekörper (10), und
- ein Verschlussteil (18), das außerhalb des Aufnahmekörpers (10) eine die Abgabeöffnung (12) freihaltende Hohlkammer (14) aufweist und ein Kopfteil (16) des Behälters bildet,
- wobei das Verschlussteil (18) mit dem Aufnahmekörper (10) über eine Trennstelle (20) lösbar verbunden ist und im Bereich der Hohlkammer (14) einander gegenüberliegende Wandteile (22) aufweist,
- **dadurch gekennzeichnet, dass** einander gegenüberliegende Wandteile (22) des Verschlussteils (18) von der Trennstelle (20) beabstandet in die Hohlkammer (14) derart hinein verdrängt sind, dass der verbleibende freie Kammerquerschnitt der Hohlkammer (14) mit seinem Aufnahmevolumen derart reduziert ist, dass das im Aufnahmekörper (10) bevorratete Abgabemedium nicht in die Hohlkammer (14) eindringen kann.

2. Behälter nach Anspruch 1, **dadurch gekennzeichnet, dass** die verdrängten Wandteile (22) des Verschlußteils (18) sich quer zu der Abgabeöffnung (12) erstrecken oder einen schrägen Winkel hierzu einnehmen.

3. Behälter nach Anspruch 1 oder 2, **dadurch gekennzeichnet, dass** die verdrängten Wandteile (22) sich innerhalb der Hohlkammer (14) des Verschlußteils (18) berühren.

4. Behälter nach Anspruch 3, **dadurch gekennzeichnet, dass** die verdrängten, einander berührenden Wandteile (22) innerhalb der Hohlkammer (14) zumindest teilweise Kammersegmente (26) freilassen, die zumindest teilweise medienführend in die Abgabeöffnung (12) münden.

5. Behälter nach einem der Ansprüche 1 bis 4, **dadurch gekennzeichnet, dass** die Hohlkammer (14) außenumfangsseitig aus Wandstücken einer Hohlkugel gebildet ist.

6. Behälter nach einem der Ansprüche 4 bis 5, **dadurch gekennzeichnet, dass** die Kammersegmente (26) insgesamt einen in sich geschlossenen Ringraum bilden, der die einander berührenden Wandteile (22) umfaßt und der in die Abgabeöffnung (12) mündet.

7. Vorrichtung zum Herstellen eines Behälters nach einem der Patentansprüche 1 bis 6 mit mindestens zwei Formbacken (28) zum Herstellen des Kopfteils (16) des Behälters, die Formausnehmungen (36) aufweisen für die Hohlkammer (14) und für die die Hohlkammer (14) begrenzenden Wandteile des Verschlußteils (18), **dadurch gekennzeichnet, dass** innerhalb der die Hohlkammer (14) betreffenden Formausnehmungen (36) ansteuerbare Formteile (38) vorhanden sind, die einander zugewandt in einer eingefahrenen Stellung die vollständige Hohlkammerbildung ermöglichen und in einer ausgefahrenen Stellung die einander benachbarten Wandteile (22) der Hohlkammer (14) formend unter Verringerung des freien Kammervolumens aufeinander zu bewegen.

8. Vorrichtung nach Anspruch 7, **dadurch gekennzeichnet, dass** das jeweilige Formteil (38) aus einem vor- und zurückfahrbaren Arbeitsstempel (40) besteht, der die jeweils ihm zuordenbare Formausnehmung (36) für die Hohlkammer (14) durchgreift, vorzugsweise in einer Ebene quer zur Längsebene der aneinander angrenzenden Formbacken (28) oder schräg dazu.

9. Vorrichtung nach Anspruch 8, **dadurch gekennzeichnet, dass** für die Verfahrbewegung des jeweiligen Arbeitsstempels (40) ein pneumatisches Ansteuermittel dient.

## Claims

1. A container, comprising:
- a hollow receptacle element (10) for holding a medium to be dispensed,
- a dispensing opening (12) formed on the receptacle element (10) for dispensing the medium to be dispensed from the receptacle element (10), and
- a sealing element (18) that has a hollow chamber (14) outside of the receptacle element (10) keeping the dispensing opening (12) clear and forms a head component (16) of the container,
- the sealing element (18) being detachably connected to the receptacle element (10) by means of a point of separation (20) and having wall components (22) lying opposite one another in the region of the hollow chamber (14),
- **characterised in that** wall components (22) of the sealing element (18) lying opposite one another, spaced apart from the point of separation (20), are displaced into the hollow chamber (14) in order to reduce the remaining free chamber cross-section of the hollow chamber (14) with its holding volume so as to prevent the medium to be dispensed stored in the receptacle element (10) from being able to penetrate into the hollow chamber (14).

2. The container according to Claim 1, **characterised in that** the displaced wall components (22) of the sealing element (18) extend transversely to the dispensing opening (12) or assume an oblique angle to the latter.

3. The container according to Claim 1 or 2, **characterised in that** the displaced wall components (22) touch within the hollow chamber (14) of the sealing element (18).

4. The container according to Claim 3, **characterised in that** the displaced wall components (22) touching each other within the hollow chamber (14) leave exposed at least partially chamber segments (26) which discharge into the dispensing opening (12), at least partially carrying media.

5. The container according to any of Claims 1 to 4, **characterised in that** on the outer peripheral side the hollow chamber (14) is formed from wall pieces of a hollow ball.

6. The container according to any of Claims 4 to 5, **characterised in that** the chamber segments (26) in their entirety form an inherently closed annular space which encompasses the wall components (22) which are touching each other and which discharges into the dispensing opening (12).

7. A device for producing a container according to any of Claims 1 to 6, comprising at least two mould jaws (28) for producing the head component (16) of the container which have mould recesses (36) for the hollow chamber (14) and for the wall components of the sealing element (18) which border the hollow chamber (14), **characterised in that** within the mould recesses (36) relating to the hollowing chamber (14) there are mould parts (38) which can be triggered, which parts facing each other in a retracted position make possible complete formation of the hollow chamber and in an extended position move the adjacent wall components (22) of the hollow chamber (14) toward each other as the free chamber volume is reduced in moulding.

8. The device according to Claim 7, **characterised in that** the respective mould part (38) consists of a plunger (40) which can move forward and backward and which passes through the mould recess (36) which may be associated to it for the hollow chamber (14), preferably in a plane transversely to the longitudinal plane of the mould jaws (28) adjacent to each other or obliquely thereto.

9. The device according to Claim 8, **characterised in that** a pneumatic triggering means is used for the traversing motion of the respective plunger (40).

## Revendications

1. Récipient comprenant :
- un corps (10) creux de réception d'un fluide à distribuer,
- une ouverture (12) de distribution, formée sur le corps (10) de réception, pour faire sortir le fluide à distribuer du corps (10) de réception, et
- une partie (18) de fermeture, qui comporte, à l'extérieur du corps (10) de réception, une chambre (14) creuse maintenant dégagée l'ouverture (12) de distribution et qui forme une partie (16) de tête du récipient,
- dans lequel la partie (18) de fermeture est reliée de manière détachable au corps (10) de réception par un point (20) destiné à se rompre et comporte des parties (22) de paroi opposées l'une à l'autre dans la zone de la chambre (14) creuse,
- **caractérisé en ce que** des parties (22) de paroi opposées l'une à l'autre de la partie (18) de fermeture sont refoulées à distance du point (20) destiné à se rompre dans la chambre (14) creuse de manière à ce que la section transversale libre restante de la chambre (14) creuse soit réduite avec son volume de réception de manière à ce que le fluide à distribuer mis en réserve dans le corps (10) de réception ne puisse pas pénétrer dans la chambre (14) creuse.

2. Récipient suivant la revendication 1, **caractérisé en ce que** les parties (22) de paroi refoulées de la partie (18) de fermeture s'étendent transversalement à l'ouverture (12) de distribution ou font un angle aigu avec elles.

3. Récipient suivant la revendication 1 ou 2, **caractérisé en ce que** les parties (22) de paroi refoulées se touchent à l'intérieur de la chambre (14) creuse de la partie (18) de fermeture.

4. Récipient suivant la revendication 3, **caractérisé en ce que** les parties (22) de paroi refoulées, qui se touchent, dégagent à l'intérieur de la chambre (14) creuse au moins en partie des segments (26) de chambre, qui débouchent au moins en partie en conduisant le fluide dans l'ouverture (12) de distribution.

5. Récipient suivant l'une des revendications 1 à 4, **caractérisé en ce que** la chambre (14) creuse est formée du côté du pourtour extérieur de pièces de paroi d'une bille creuse.

6. Récipient suivant l'une des revendications 4 à 5, **caractérisé en ce que** les segments (26) de chambre forment dans l'ensemble un espace annulaire fermé en soi, qui comprend des parties (22) de paroi se touchant et qui débouche dans l'ouverture (12) de distribution.

7. Dispositif de fabrication d'un récipient suivant l'une des revendications 1 à 6, comprenant au moins deux mâchoires (28) de moule pour la fabrication de la partie (16) de tête du récipient, qui ont des évidements (36) de moule pour la chambre (14) creuse et pour les parties de paroi de la partie (18) de fermeture, qui limitent la chambre (14) creuse, **caractérisé en ce qu'**il y a, à l'intérieur des évidements (36) de moule concernant la chambre (14) creuse, des parties (38) de moule, pouvant être réglées, qui rendent possible, dans une position rentrée, en étant tournées l'une vers l'autre, la formation complète de la chambre creuse et, dans une position sortie, déplacent l'une vers l'autre les parties (22) de paroi voisines de la chambre (14) creuse avec moulage en diminuant le volume libre de la chambre.

8. Dispositif suivant la revendication 7, **caractérisé en ce que** la partie (38) de moule respective est constituée d'un poinçon (40) de travail allant et revenant, qui pénètre dans l'évidement (36) de moule qui lui est associé respectivement pour la chambre (14) creuse, de préférence dans un plan transversal au plan longitudinal des mâchoires (28) de moule voisines l'une de l'autre ou en étant incliné par rapport à elles.

9. Dispositif suivant la revendication 8, **caractérisé en ce que** l'on se sert d'un moyen de commande pneumatique pour le déplacement du poinçon (40) de travail respectif.
